# EUROPÄISCHE PATENTANMELDUNG

(11) **EP 1 331 231 A2**
(43) Veröffentlichungstag der Anmeldung: **30.07.2003**
(21) Anmeldenummer: 03000713.2
(22) Anmeldetag: 13.01.2003
(51) Int. Cl.: C08F 20/34, C08F 8/30, A01N 33/12, C09D 5/14, C07C 219/08

(54) **Polymere mit biozider Wirkung, Verfahren zu ihrer Herstellung und ihre Verwendung**

(30) Priorität: 29.01.2002 DE 10203342
(71) Anmelder: Clariant GmbH, 65929 Frankfurt am Main (DE)
(72) Erfinder: Crass, Gerhard, Dr., 61169 Friedberg (DE); Falk, Uwe, Dr., 63486 Bruchköbel (DE); Ivan, Béla, Prof. Dr., 1038 Budapest (HU); Erdödi, Gabor, 8047 Csokako (HU); Màthé, Arpàd, Dr., 1125 Budapest (HU)
(74) Vertreter: Mikulecky, Klaus, Dr.

(57) **Zusammenfassung**

Gegenstand der Erfindung sind Polymere, bestehend im wesentlichen aus Struktureinheiten der Formel (1) 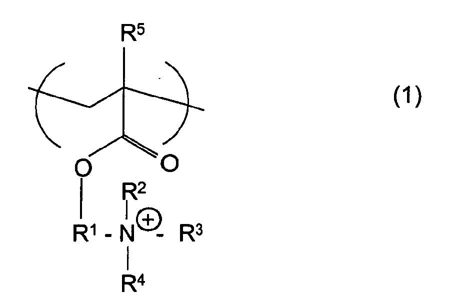 worin
- R¹: eine C₁-C₄-Alkylengruppe
- R², R³: unabhängig voneinander eine C₈-C₁₂-Alkyl- oder Alkenylgruppe
- R⁴: Methyl oder Ethyl
- R⁵: Wasserstoff oder Methyl
bedeuten und deren Molekulargewicht zwischen 1000 und 100.000 g/mol beträgt, Zwischenprodukte für ihre Herstellung, ein Verfahren zu ihrer Herstellung sowie ihre Verwendung als biozides Beschichtungsmittel für Oberflächen und Textilien.

## Beschreibung

### Polymere mit biozider Wirkung, Verfahren zu ihrer Herstellung und ihre Verwendung

Die vorliegende Erfindung betrifft Polymere aus N,N,N-Trialkylammoniumalkyl-(meth)acrylaten, ein Verfahren zu ihrer Herstellung und ihre Verwendung zur bioziden Ausrüstung von Oberflächen oder Textilien.

Es kann aus verschiedenen Gründen erforderlich sein, harten Oberflächen oder Textilien eine biozide Ausrüstung zu geben. Hierdurch soll es vermieden werden, dass sich auf diesen genannten Substraten Mikroorganismen ansiedeln können, und zwar ohne deren wiederholte Desinfektion durch biozide Mittel.

Beschichtungsmittel für harte Oberflächen und Textilien sind im Prinzip bekannt.

So offenbart DE-A-100 62 355 Copolymere zur Beschichtung harter Oberflächen oder von Textilien, die anionische Vinylmonomere und quartäre Ammoniumacrylate als Comonomere umfassen. Eine biozide Wirkung solcher Copolymere wird nicht offenbart.

DE-A-199 21 894 offenbart ein Verfahren zur bioziden Ausrüstung von Oberflächen, bei dem Polymere aus mindestens einfach aliphatisch ungesättigten Monomeren der allgemeinen Formel

R¹NRₐR_{b}³R_{c}⁴X_{d}

mit
R¹ = H, verzweigter, unverzweigter oder cyclischer, gesättigter oder ungesättigter Kohlenwasserstoffrest mit bis zu 50 C-Atomen, der durch O-, N- oder S-Atome substituiert sein kann,
R², R³, R⁴ = H, verzweigter, unverzweigter oder cyclischer gesättigter oder ungesättigter Kohlenwasserstoffrest mit bis zu 25 C-Atomen, der durch O-, N- oder S-Atome substituiert sein kann, wobei R², R³, R⁴ gleich oder verschieden sind,
X: ein Anion,
a, b, c, d: jeweils 0 oder 1
zur Anwendung kommen. Als Beispiele für quartäre Stickstoffgruppen enthaltende Monomere werden 2-Methacryloyloxyethyl-trimethylammoniumchlorid, 2-Methacryloyloxyethyl-trimethylammoniumsulfat,
3-Methacryloylaminopropyl-trimethylammoniumchlorid, N,N,N-trimethyl-3-(2-methyl-1-oxo-2-propenylamino)-1-propanammoniumchlorid; N,N,N-triethyl-2-(1-oxo-2-propenylamino)-ethanammonium, N,N,N-trimethyl-ethenammoniumbromid genannt.

DE-A-199 21 904 offenbart Polymere, die zur bioziden Ausrüstung von Oberflächen geeignet sind. Diese müssen aus Monomeren aufgebaut sein, welche wenigstens eine quartäre Aminogruppe enthalten. Das Dokument nennt eine Reihe geeigneter Monomere mit Methyl-, Ethyl- oder Benzylsubstitution am Stickstoffatom.

Es wurde erkannt, dass die polymeren Beschichtungsmittel des Standes der Technik in ihrer bioziden Wirkung verbesserungsbedürftig sind.

Der Erfindung lag daher die Aufgabe zugrunde, neue Polymere aufzufinden, die auf Oberflächen oder Textilien aufgebracht werden können, und dort eine biozide Wirkung entfalten.

Überraschenderweise wurde gefunden, dass Polymere aus N,N,N-Trialkylammoniumalkyl(meth)acrylaten diese geforderten Eigenschaften aufweisen, sofern sie lange Alkylketten tragen.

Gegenstand der Erfindung sind daher Polymere, bestehend im wesentlichen aus Struktureinheiten der Formel (1) 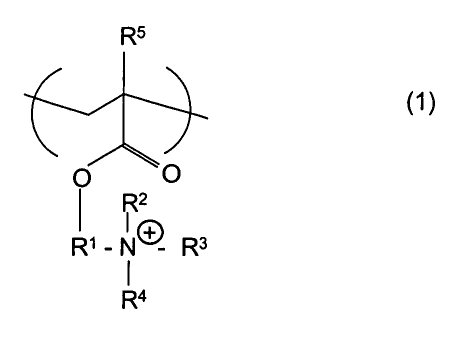 worin
- R¹: eine C₁-C₄-Alkylengruppe
- R², R³: unabhängig voneinander eine C₈-C₁₂-Alkyl- oder Alkenylgruppe
- R⁴: Methyl oder Ethyl
- R⁵: Wasserstoff oder Methyl
bedeuten und deren Molekulargewicht zwischen 1000 und 100.000 g/mol beträgt.

Ein weiterer Gegenstand der Erfindung sind Polymere, bestehend im wesentlichen aus Struktureinheiten der Formel (2) 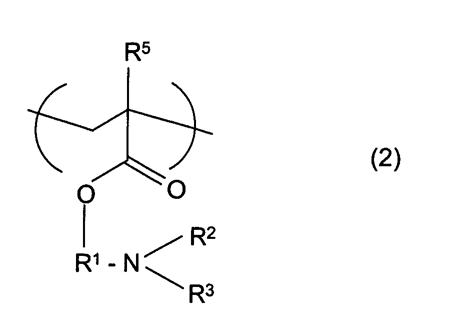 worin R¹, R², R³ und R⁵ die oben angegebene Bedeutung haben, und die ein Molekulargewicht von 1000 bis 100.000 g/mol aufweisen.

Der Begriff "im wesentlichen" bedeutet hier, dass die erfindungsgemäßen Polymere keine weiteren Struktureinheiten, die die Eigenschaften der Polymere merklich beeinflussen außer denen enthalten, die den Formeln 1 und 2 entsprechen. Es wird davon ausgegangen, dass die Polymere vorzugsweise maximal 2 Mol-%, insbesondere maximal 0,5 Mol-% weitere Struktureinheiten außer Formeln 1 und 2 enthalten können, ohne ihre Eigenschaften merklich zu ändern.

Die Polymere der Formel 1 sind aus den Polymeren der Formel 2 durch Quaternierung herstellbar. Die Polymere der Formel 2 stellen daher ein Zwischenprodukt für die Polymere der Formel 1 dar.

Ein weiterer Gegenstand der Erfindung ist ein Verfahren zur Herstellung von Polymeren der Formel 1, indem Polymere der Formel 2 mit einem Methylierungsoder Ethylierungsmittel umgesetzt werden.

Ein weiterer Gegenstand der Erfindung ist ein Verfahren zur Herstellung von Polymeren der Formel 1, indem man einen (Meth)acrylsäurealkylester, vorzugsweise einen Methylester, mit einer Verbindung der Formel 3 zum entsprechenden (Meth)acrylsäureester umestert, daran anschließend dieses umgeesterte Produkt mit einem Methylierungs- oder Ethylierungsmittel zur quaternären Ammoniumverbindung alkyliert, und diese quaternäre Ammoniumverbindung der radikalischen Polymerisation unterzieht.

Ein weiterer Gegenstand der Erfindung ist ein Verfahren zur Herstellung von Polymeren der Formel 2 durch entweder
A) Umsetzung einer Verbindung der Formel 3

   HO - R¹ - NR²R³ (3)

   mit (Meth)acrylsäurealkylester in Gegenwart eines Veresterungskatalysators unter Abdestillieren des bei der Reaktion entstehenden Alkanols,und anschließende radikalische Polymerisation des erhaltenen Produkts,
   oder
B) Umsetzung eines Polymethacrylsäurealkylesters mit einer Verbindung der Formel 3 in Gegenwart eines Veresterungskatalysators unter Abdestillieren des bei der Reaktion entstehenden Alkanols.

Folgt man der oben beschriebenen Variante A), so erhält man einen (Meth)acrylsäureester der Formel 4 als Zwischenprodukt:

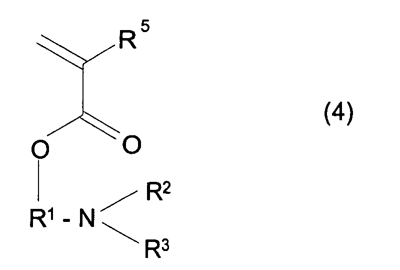

Ein weiterer Gegenstand der Erfindung ist ein Verfahren zur Herstellung von Polymeren der Formel 1 durch Umsetzung einer Verbindung der Formel 3 zum entsprechenden (Meth)acrylsäureester, anschließender Quaternierung des erhaltenen (Meth)acrylsäureesters, und anschließender radikalischer Polymerisation des erhaltenen quaternierten (Meth)acrylsäureesters.

Der quaternierte (Meth)acrylsäureester entspricht der Formel 5. Die Verbindungen der Formel 5 sind ein weiterer Gegenstand der Erfindung. Die Reste R¹, R², R³, R⁴ und R⁵ haben die oben angegebene Bedeutung.

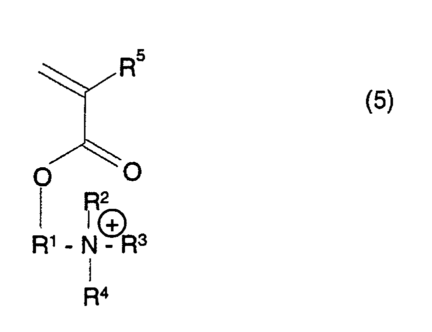

Die Umsetzung von Verbindungen der Formel 3 zum (Meth)acrylsäureester kann mit (Meth)acrylsäurealkylestern, vorzugsweise (Meth)acrylsäuremethylester, erfolgen.

Vorzugsweise findet ein Veresterungskatalysator, insbesondere Alkalihydroxide, Alkalicarbonate, Erdalkalihydroxide und organische Zinn-, oder Titanverbindungen Verwendung. Die entstehenden (Meth)acrylsäureester entsprechen der Formel 4.

Ein weiterer Gegenstand der Erfindung ist die Verwendung von Polymeren der Formel 1 zur bioziden Ausrüstung von Oberflächen und Textilien.

R¹ steht vorzugsweise für einen Ethylen- oder Propylenrest, insbesondere für einen Ethylenrest.

R² und R³ stehen vorzugsweise beide für den gleichen Rest. Es ist insbesondere bevorzugt, dass R² und R³ für Octyl- oder Decylreste, speziell Decylreste stehen.

R⁴ steht vorzugsweise für einen Methylrest.

R⁵ steht vorzugsweise für einen Methylrest.

Bei einer besonders bevorzugten Ausführungsform handelt es sich bei den erfindungsgemäßen Polymeren um solche, in denen R¹ = Ethylen, R² = R³ = Decyl, R⁴ = R⁵ = Methyl bedeuten.

Die Molekulargewichte der erfindungsgemäßen Polymere der Formel 1 liegen vorzugsweise zwischen 2000 und 60.000 g/mol, insbesondere zwischen 5000 und 40.000 g/mol.

Die Quaternierung der Stickstoffatome sowohl in den Polymeren nach Formel 2 zu den Polymeren nach Formel 1 als auch in den Monomeren wird vorzugsweise mit einem Alkylsulfat, oder einem Alkylhalogenid durchgeführt. Besonders bevorzugt sind Dimethylsulfat oder Methylchlorid.

Trägt das Stickstoffatom nach der Quaternierung eine positive Ladung, so ist ein entsprechendes Gegenion vorhanden. Gegenionen können beispielsweise F⁻, Cl⁻, Br⁻, I⁻, SO₄²⁻, CH₃SO₃⁻, CH₃CO₂⁻, C₂O₄²⁻, CO₃²⁻, PO₄³⁻, PO₃²⁻, NO₃⁻, NO₂⁻, NO⁻, CN⁻, SCN⁻, CNO⁻, ClO⁻, ClO₂⁻, ClO₃⁻, ClO₄⁻ sein.

Die Umesterung des (Meth)acrylsäuremethylesters mit der Verbindung der Formel 3 wird vorzugsweise in Gegenwart eines basischen Veresterungskatalysators durchgeführt. Bevorzugte Katalysatoren sind Alkalihydroxide, Alkalicarbonate, Erdalkalihydroxide und organische Zinn- oder Titanverbindungen. Besonders bevorzugt sind Sn(C₄H₉)₄, Sn(C₄H₉)O₂, Sn(C₄H₉)Cl₂, Ti(i-C₃H₇)₄, Ti(C₂H₅)₄, K₂CO₃, KOH, Ca(OH)₂, NaOH, NaOCH₃, Li₂CO₃ und LiOH.

Die radikalische Polymerisation der aus einer Umsetzung von (Meth)acrylsäuremethylester und der Verbindung der Formel 3 erhaltenen Verbindung erfolgt nach den im Stand der Technik bekannten Verfahren.

Als Initiator für das erfindungsgemäße Verfahren kommen die im Stand der Technik bekannten Initiatoren für Radikalpolymerisationen in Betracht, bevorzugt ist Azoisobutyronitril. Das Gewichtsverhältnis von Verbindungen der Formel 2 zum Initiator liegt vorzugsweise unterhalb von 600:1, insbesondere unterhalb von 250:1.

Die Polymerisation wird vorzugsweise bei Temperaturen zwischen 50 und 80°C, insbesondere 55 bis 70°C durchgeführt. Die mindestens einmalige Steigerung der Reaktionstemperatur um mindestens 10°C erfolgt vorzugsweise nach 40 % der gesamten Reaktionsdauer, insbesondere nach 50 % der gesamten Reaktionsdauer.

Die Polymerisation kann in einem Lösemittel durchgeführt werden. Beispiele geeigneter Lösemittel sind niedere Alkohole und niedere Alkylbenzole, vorzugsweise Methanol oder Toluol. Die Polymerisation kann auch in Abwesenheit von Lösemitteln durchgeführt werden.

Wird ein Lösemittel verwendet, so betragen die Konzentrationen der Monomere vorzugsweise etwa 30 bis 50 Gew.-%.

Zur Anwendung als biozides Ausrüstungsmittel wird das Polymer nach Formel 1 in alkoholischer und/oder wässriger Lösung mit einem Gehalt von 1 - 50 g/l auf die zu behandelnden Oberflächen und Textilien aufgebracht. Die Polymere nach Formel 1 sind insbesondere für die Ausrüstung von Wolle, Polyester, Polyamid und Baumwolle geeignet.

### Beispiele

### A) Umesterung von Methacrylsäuremethylester (MMA) mit Didecylaminoethanol (DDAE)

Zur Umesterung von MMA mit DDAE wurden diese Stoffe mit einem Katalysator gemischt und bei erhöhter Temperatur und vermindertem Druck zur Reaktion gebracht. Während der Reaktion wurde das entstehende Methanol abdestilliert. Im folgenden (Tabelle 1) wird der Einfluss des Katalysators auf die Ausbeute der Umesterungsreaktion gezeigt

**Tabelle 1:**

| Umesterung von MMA mit DDAE | | |
|---|---|---|
| Beispiel | Katalysator | Ausbeute, % |
| 1 | Al(iPrO)₃ | ∼ 0 |
| 2 | Ti(PrO)₄ | 77 |
| 3 | K₂CO₃ | 12 |
| 4 | K₂CO₃ | 4 |
| 5 | K₂CO₃ | 4 |
| 6 | Ca(OH)₂ | 6 |
| 7 | LiAlH₄ | 50 |
| 8 | Dibutylzinnmaleat (DBTM) | 65 |
| 9 | DBTM | 51 |
| 10 | DBTM | 85 |
| 11 | DBTM | 57 |
| 12 | DBTM | 60 |
| 13 | Sn(Bu)₄ | 2 |
| 14 | Sn(Bu)₄ | 0 |
| 15 | K₂CO₃ (Pulver) | 2 |
| 16 | K₂CO₃ (Pulver) + PEG | 4 |
| 17 | KOH | 5 |
| 18 | Ca(OH)₂ | 1 |
| 19 | NaOH + PEG | 35 |
| 20 | NaOH | 36 |
| 21 | NaOCH₃ | ∼ 0 |
| 22 | Li₂CO₃ | 3 |
| 23 | LiOH | 66 |
| 24 | LiOH + PEG | 68 |
| 25 | LiOH | 65 |
| 26 | LiOH | 72 |
| 27 | LiOH | 62 |
| 28 | LiOH | 68 |
| 29 | LiOH | 58 |
| 30 | LiOH | 55 |
| 31 | LiOH | 51 |
| 32 | LiOH | 62 |

### B) Umesterung von Polymethacrylsäuremethylester (PMMA) mit DDAE

Die polymeranaloge Umesterung wurde wie die monomere Umsetzung unter A) durchgeführt. Die Reaktanden wurden in Totuol gelöst, nach der Reaktion wurde das Produkt mit Methanol gefällt und im Vakuum getrocknet. RT bedeutet Reaktionstemperatur, RZ bedeutet Reaktionszeit.

**Tabelle 2**

| Beispiel | g DDAE | g PMMA | RT, °C | RZ, h | Katalysator |
|---|---|---|---|---|---|
| 33 | 10,76 | 1,31 | 100 | 10 | KOH |
| 34 | 10,76 | 1,31 | 160 | 10 | LiACH₄ |
| 35 | 17,0 | 2,07 | 100 | 10 | Dibutylzinnmaleat |

### C) Quaternierung des DDAEMA

### Beispiel 36

300 g DDAEMA und 0,07 g p-Methoxyphenol wurden in einem Autoklaven auf 120°C erwärmt. Es wurde Methylchlorid bis zu einem Druck von 4 bar aufgepreßt. Der Druck wurde durch nachdosieren bei 4 bar gehalten. Nach 12 h Reaktionszeit wurde der Autoklav entspannt und das überschüssige Methylchlorid im Vakuum entfernt. Man erhielt 320 g des quaternierten Produkts. Der Quaternierungsgrad betrug 80%.

### Beispiel 37

100 g DDAEMA und 0,02 g p-Methoxyphenol wurden auf 40°C erwärmt. Im Laufe von 15 Minuten wurden 31 g Dimethylsulfat zugetropft und die Reaktionsmischung noch 10 h bei 40°C gerührt. Man erhielt 125 g des quaternierten Produkts. Der Quaternierungsgrad betrug 97%

### D) Polymerisation des Didecylaminoethylmethacrylats (DDAEMA)

Die Polymerisation des DDAEMA wurde durch Mischen dieses Stoffs mit AIBN als Radikalstarter und nachfolgende Entgasung unter reduziertem Druck eingeleitet. Die Polymerisation wurde unter Argonatmosphäre durchgeführt, das Produkt wurde mit Methanol gefällt.

**Tabelle 3**

| Beispiel | DDAEMA aus Beispiel | RT, °C | RZ, h |
|---|---|---|---|
| 38 | 8,5 g aus Beispiel 1 | 75 | 6 |
| 39 | 56,2 g aus Beispiel 12 | 75 | 2 |

### E) Herstellung von quaterniertem Poly-N,N-didecylaminoethylmethacrylat

### 1. Herstellung von N,N-Didecylaminoethylmethacrylat (DDAEMA) (entspricht Beispiel 32)

257,4 g DDAE, 1,508 g Phenothiazin und 736,4 mg LiOH wurden unter Stickstoffatmosphäre bei Normaldruck in einem geheizten Reaktionsgefäß für 30 Minuten gerührt. Dann wurden 150,7 g MMA (zweifacher molarer Überschuss) bei einer Ansatztemperatur von 90°C zugegeben. 30 Minuten später war die Temperatur der Reaktion auf 104°C angestiegen und die Destillation des entstehenden Methanols setzte ein. Der Druck wurde innerhalb von 60 min auf 126 Torr reduziert, später auf 24 Torr. Verbleibendes MMA wurde bei diesem Druck abdestilliert. Es wurden 292,5 g DDAEMA erhalten.

Inhibitor und LiOH wurden mit 20 g Al₂O₃ aus dem Destillationsrückstand entfernt. Das Produkt wurde innerhalb 1 Tag durch eine Al₂O₃-Chromatographiesäule ohne Druck gereinigt.

### 2. Polymerisation des DDAEMA zu Poly-DDAEMA

### Beispiel 40

50 g des Produkts aus Beispiel 32 (enthält 35,5 g DDAEMA), 142 g AIBN und 50 cm³ Hexan wurden in einem Reaktionsgefäß gemischt. Die Lösung wurde auf -20°C abgekühlt und es wurde Argon für 15 min durchgeleitet. Zum Einleiten der Polymerisation wurde die Temperatur dann auf 65°C erhöht. 16 Stunden später wurde das Reaktionsgemisch auf Raumtemperatur abgekühlt, und das Produkt in 1 I Methanol gefällt. Die Fällung wurde dann in 200 ml Hexan aufgenommen und erneut mit 500 ml Methanol gefällt. Das so erhaltene Polymer wurde in 200 ml Hexan gelöst und mit Wasser extrahiert, um Methanolreste zu entfernen. Danach wurde die Hexanlösung über CaCl₂ getrocknet, filtriert und das Hexan wurde abgezogen. Es wurden 24,3 g Poly-DDAEMA erhalten.

### 3. Quaternierung des Poly-DDAEMA

Zur Quaternierung des Poly-DDAEMA wurde eine bekannte Menge von 14 g gelöst in Hexan, des Produkts aus Beispiel 40 entnommen, und mit 20 ml Hexan oder 10 ml Hexan und 20 mol Isobutanol verdünnt. Das Reaktionsgefäß wurde dann in ein Isopropanol/Trockeneisbad gestellt und es wurde eine Lösung von Dimethylsulfat in Isobutanol zugegeben. Die Reaktionstemperatur wurde bei ca. 20°C gehalten. Nach der Reaktion wurden die Lösungsmittel abgezogen und das quaternierte Polymer isoliert, und getrocknet.

### Beispiel 47

40 g des Poly-DDAEMA wurden in 217 g n-Hexan und 10 g i-Propanol gelöst und auf 60°C erwärmt. Im Laufe von 15 Minuten wurden 12,3 g Dimethylsulfat zugetropft und die Reaktionsmischung noch 10 h bei 60°C gerührt. Die Lösungsmittel wurden im Vakuum abdestilliert und man erhielt 48 g des quaternierten Polymers. Der Quaternierungsgrad betrug 97 %.

### Beispiel 48

38 g des Poly-DDAEMA wurden in 195 g n-Hexan und 10 g i-Propanol gelöst und in einem Autoklaven auf 66°C erwärmt. Es wurde Methylchlorid bis zu einem Druck von 4 bar aufgepreßt. Der Druck wurde durch nachdosieren bei 4 bar gehalten. Nach 6 h Reaktionszeit wurde der Autoklav entspannt und das überschüßige Methylchlorid und die Lösungsmittel im Vakuum entfernt. Man erhielt 35 g des quaternierten Polymers. Der Quaternierungsgrad betrug 95 %.

**Tabelle 4:**

| Quaternierung von Poly-DDAEMA | | | | |
|---|---|---|---|---|
| Beispiel | Menge Poly-DDAEMA, g | Menge Quat. g | Quaternierungsgrad, % | Reaktionszeit, min |
| 41 | 14,089 | 3,04 | 100 | 40 |
| 42 | 13,357 | 3,004 | 100 | 60 |
| 43 | 14,196 | 3,018 | 82 | 20 |
| 44 | 13,82 | 2,847 | 63 | 15 |
| 45 | 13,258 | 2,589 | 44 | 15 |
| 46 | 13,271 | 2,508 | 22 | 28 |
| 47 | 40 | 48 | 97 | 615 |
| 48 | 38 | 35 | 95 | 360 |

### F) Polymerisation des quaternierten DDAEMA

### Beispiel 49

50 g des Produkts aus Beispiel 36 (enthält 47,5 g methyl-quaterniertes DDAEMA), 145 g AIBN und 55 cm³ Hexan wurden in einem Reaktionsgefäß gemischt. Die Lösung wurde auf -20°C abgekühlt und es wurde Argon für 15 min durchgeleitet. Zum Einleiten der Polymerisation wurde die Temperatur dann auf 65°C erhöht. 16 Stunden später wurde das Reaktionsgemisch auf Raumtemperatur abgekühlt, und das Produkt in 1 I Methanol gefällt. Die Fällung wurde dann in 200 ml Hexan aufgenommen und erneut mit 500 ml Methanol gefällt. Das so erhaltene Polymer wurde in 200 ml Hexan gelöst und mit Wasser extrahiert, um Methanolreste zu entfernen. Danach wurde die Hexanlösung über CaCl₂ getrocknet, filtriert und das Hexan wurde abgezogen. Es wurden 26,8 g Poly-(methyl-quaterniertes) DDAEMA erhalten.

Die biozide Wirkung der erfindungsgemäßen quaternierten Polymere wurde mit Hilfe der shake-flask-Methode ermittelt.

Diese Prüfmethode dient zur quantitativen Bestimmung der antibakteriellen Wirkung von nicht diffundierenden Wirkstoffen. Die Methode hat gegenüber anderen den Vorteil, dass sie einen guten Kontakt zwischen Bakterien und ausgerüstetem Substrat gewährleistet. Für die Untersuchung eignen sich Fasern und Gewebe.

Die Prüfmuster von 0,75 g werden in eine 100 ml Pyrex-Flasche mit 70 ml steriler Pufferlösung gegeben. Als Pufferlösung dient eine Lösung von 3,4 g KH₂PO₄ in 100 ml H₂O, die mit NaOH auf pH 7,2 eingestellt ist. Diese Gebrauslösung wird 1:800 verdünnt.

Zur Prüfung wird die Pufferlösung mit 5 ml Inokulum versetzt und kurz geschüttelt. Zur Herstellung des Inokulums wird die Tageskultur mit Puffer-Lösung so verdünnt, dass die Endkonzentration bei ca. 1-3 x 10⁵ KbE/ml (Kolonienbildende Einheiten/ml) liegt.

Aus einer Flasche wird sofort die Keimzahl pro ml für 0 Stunden bestimmt, durch Ausplattung (10¹, 10⁻¹, 10⁻², 10⁻³) auf TSA (Trypticase Soy Agar).

Die Flaschen werden 24 Stunden bei Raumtemperatur auf der Schüttelmaschine geschüttelt. Danach wird aus jeder Flasche die Keimzahl pro ml für 24 Stunden bestimmt, durch Ausplattung (10¹, 10⁻¹, 10⁻², 10⁻³) auf TSA. Es wird wie oben bei 0 Stunden vorgegangen.

Die Beurteilung erfolgt über die Auszählung der "Kolonienbildenden Einheiten" nach 0 Stunden und nach 24 Stunden Schüttelzeit der Muster. Die resultierenden Zahle der Kolonienbildenden Einheiten (KbE) werden in logarithmischer Form angegeben und nach folgender Formel berechnet:
log (Reduktion) = (log KbE/ml nach 0 Stunden Kontaktzeit) - (log KbE/ml nach 24 Stunden Kontaktzeit)

Die Bewertung des Ergebnisses ist wie folgt:
- log Reduktion < 0,0 :: keine antibakterielle Wirkung
- log Reduktion 0,1 - 1,0 :: bakteriostatische Wirkung
- log Reduktion 1,1 - 2,0 :: bakterizide Wirkung
- log Reduktion > 2,0 :: signifikante bakterizide Wirkung

Als Tageskultur wurde Staphylococcus Aureus ATCC 6538 verwendet.

**Tabelle 5:**

| Wirksamkeit der erfindungsgemäßen quaternierten Poly-DDAEMA | | | |
|---|---|---|---|
| Beispiel | Muster | quat. Poly-DDAEMA | log (Reduktion) |
| 50 | ohne | - | - 0,2 |
| 51 | Baumwolle | 0 | - 0,1 |
| 52 | Baumwolle | 10 | - 0,2 |
| 53 | Baumwolle | 20 | 0,9 |
| 54 | Wolle | 20 | 2,1 |
| 55 | Wolle | 20 | > 4,3 |
| 56 | Polyester | 10 | > 4,3 |
| 57 | Polyester | 20 | > 4,3 |
| 58 | Polyamid | 10 | 4,0 |
| 59 | Polyamid | 20 | > 4,3 |

## Patentansprüche

1. Verwendung von Polymeren bestehend im wesentlichen aus Struktureinheiten der Formel 1 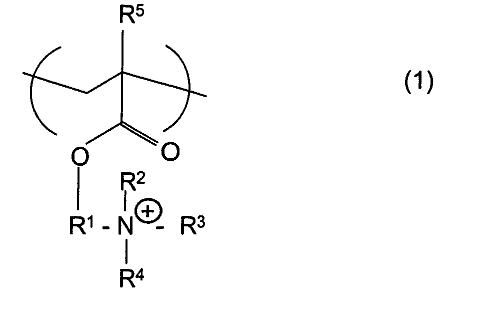 worin
R¹ eine C₁-C₄-Alkylengruppe
R², R³ unabhängig voneinander eine C₈-C₁₂-Alkyl- oder Alkenylgruppe
R⁴ Methyl oder Ethyl
R⁵ Wasserstoff oder Methyl
bedeuten und deren Molekulargewicht zwischen 1000 und 100.000 g/mol beträgt, zur bioziden Ausrüstung von Oberflächen und Textilien.

2. Verwendung gemäß Anspruch 1, worin R¹ für einen Ethylen- oder Propylenrest steht.

3. Verwendung gemäß Anspruch 1 und/oder 2, worin R² und R³ beide für Octyloder Decylreste stehen.

4. Verwendung gemäß einem oder mehreren der Ansprüche 1 bis 3, worin R⁴ für einen Methylrest steht.

5. Verwendung gemäß einem oder mehreren der Ansprüche 1 bis 4, worin R⁵ für einen Methylrest steht.

6. Verwendung gemäß einem oder mehreren der Ansprüche 1 bis 5, worin das Molekulargewicht der Polymeren zwischen 2000 und 60.000 g/mol liegt.

7. Verfahren zur Herstellung von Polymeren bestehend im wesentlichen aus Struktureinheiten der Formel 2 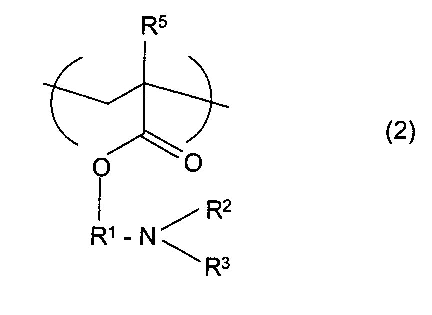 worin R¹, R², R³ und R⁵ die in Anspruch 1 angegebene Bedeutung haben, und die ein Molekulargewicht von 1000 bis 100.000 g/mol aufweisen, durch entweder
A) Umsetzung einer Verbindung der Formel 3
HO - R¹ - NR²R³ (3)
mit (Meth)acrylsäurealkylester in Gegenwart eines Veresterungskatalysators unter Abdestillieren des bei der Reaktion entstehenden Alkanols,und anschließende radikalische Polymerisation des erhaltenen Produkts,
oder
B) Umsetzung eines Polymethacrylsäurealkylesters mit einer Verbindung der Formel 3 in Gegenwart eines Veresterungskatalysators unter Abdestillieren des bei der Reaktion entstehenden Alkanols.

8. Verfahren nach Anspruch 7, wobei Alkalihydroxide, Alkalicarbonate, Erdalkalihydroxide und organische Zinn-, oder Titanverbindungen als Katalysatoren verwendet werden.

9. Verfahren zur Herstellung von Polymeren der Formel 1, indem Polymere der Formel 2 mit einem Methylierungs- oder Ethylierungsmittel umgesetzt werden.

10. Verfahren zur Herstellung von Polymeren der Formel 1 durch
a) Umsetzung einer Verbindung der Formel 3 zum entsprechenden (Meth)acrylsäureester,
b) anschließender Quaternierung des erhaltenen (Meth)acrylsäureesters, und
c) anschließender radikalischer Polymerisation des erhaltenen quaternierten (Meth)acrylsäureesters.

11. Polymere, bestehend im wesentlichen aus Struktureinheiten der Formel (1) 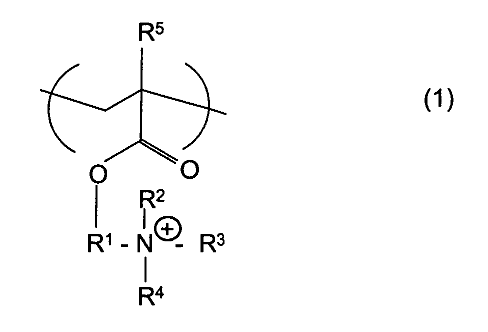 worin
R¹ eine C₁-C₄-Alkylengruppe
R², R³ unabhängig voneinander eine C₈-C₁₂-Alkyl- oder Alkenylgruppe
R⁴ Methyl oder Ethyl
R⁵ Wasserstoff oder Methyl
bedeuten und deren Molekulargewicht zwischen 1000 und 100.000 g/mol beträgt.

12. Polymere, bestehend im wesentlichen aus Struktureinheiten der Formel (2) 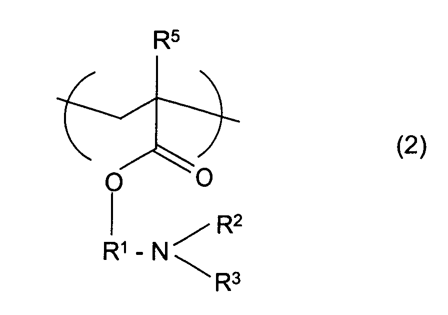 worin R¹, R², R³ und R⁵ die in Anspruch 11 angegebene Bedeutung haben, und die ein Molekulargewicht von 1000 bis 100.000 g/mol aufweisen.

13. Verbindungen der Formel 5 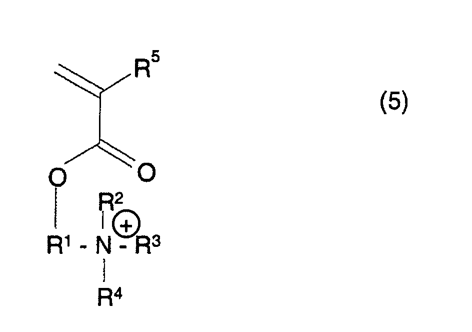 worin die Reste R¹, R², R³, R⁴ und R⁵ die in Anspruch 11 angegebene Bedeutung haben.
